# EUROPÄISCHE PATENTSCHRIFT

(11) **EP 2 211 961 B1**
(45) Veröffentlichungstag und Bekanntmachung des Hinweises auf die Patenterteilung: **10.12.2014**
(21) Anmeldenummer: 08842359.5
(22) Anmeldetag: 23.10.2008
(51) Int. Cl.: A61M 16/04, A61M 16/08, A61M 39/10

(54) **ADAPTER ZUM ANSCHLUSS EINER INSBESONDERE RESPIRATORISCHEN HILFSEINRICHTUNG AN EINEN TUBUS**
ADAPTER FOR CONNECTING PARTICULARLY A RESPIRATORY IMPLEMENT TO A TUBE
ADAPTATEUR DESTINÉ AU RACCORDEMENT NOTAMMENT D'UN DISPOSITIF AUXILIAIRE RESPIRATOIRE À UN TUBE

(30) Priorität: 25.10.2007 DE 102007050974
(43) Veröffentlichungstag der Anmeldung: 04.08.2010
(73) Patentinhaber: medi1one medical gmbh, 71336 Waiblingen (DE)
(72) Erfinder: FRICK, Ulrich, 71404 Korb (DE); KAUFFMANN, Albrecht, 71384 Weinstadt (DE)
(74) Vertreter: Kesselhut, Wolf
(86) Internationale Anmeldenummer: PCT/EP2008/008981
(87) Internationale Veröffentlichungsnummer: WO 2009/053074

(56) Entgegenhaltungen:
- WO-A2-2007/092199
- DE-A1- 1 440 918
- US-A- 4 729 765
- US-A1- 2008 041 391

## Beschreibung

Die Erfindung betrifft einen Adapter zum Anschluss einer insbesondere respiratorischen Hilfseinrichtung wie z.B. einem Schlauch, eine Tubus-Verlängerung, einen Atemluftfilter, einen Beatmungsbeutel oder eine künstlichen Nase oder dergleichen, an einen in den menschlichen Körper einsetzbaren Tubus mit einem sich bevorzugt konisch verjüngenden Anschlusskonnektor, gemäß dem Oberbegriff von Anspruch 1.

Bei den bekannten Anschlussadaptern, mit denen sich die zuvor genannten Atemluftzufuhr- oder Aufbereitungseinrichtungen - die nachfolgend der Einfachheit halber als respiratorische Hilfseinrichtungen bezeichnet werden - an die Anschlusskonnektoren von Tuben anschließen lassen, ergibt sich das Problem, dass die in der Regel leicht konisch zusammen laufenden Anlageflächen der Anschlusskonnektoren nach dem Zusammenstecken durch das Adhäsionsverhalten des Kunststoffmaterials und die Konizität der Kontaktflächen bedingt vergleichsweise hohe Losbrechmomente benötigen, um die reibschlüssig dichtende Klemmverbindung wieder zu lösen.

Durch diese vergleichsweise hoben Kräfte und Drehmomente, die zum Lösen der Verbindung zwischen Adapter und Anschlusskonnektor überkommen werden müssen ergibt sich das Problem, dass z.B. ein in den Halsbereich eines Patienten eingesetzter Trachealtubus häufig ungewollt im Halsbereich bewegt wird, was beim Patienten zu Schmerzen und im Laufe der Zeit auch zu Verletzungen führen kann.

Die DE-OS 1440918 besehreibt eine schnell lösbare Kupplung für eine elektrische Steckverbindung, die einen weiblichen Aufnahmsverhinderteil und einen in diesen Aufnahmeverbinderteil einsteckbaren männlichen Steckverbinderteil aufweiset. An letzterem ist ein langgestreckter Steckkörper mit kreisförmigem Querschnitt ausgeformt, dessen Durchmesser im Wesentlichen dem Innendurchmesser der Bohrung im weiblichen Aufnahmeverbinderteil entspricht, sodass der Steckkörper in die Bohrung einsteckbar ist, um einen Kontakt zwischen den im Inneren des Steckverbinderteils und des Aufnahmeverbinderteils angeordneten elektrischen Steckverbindungen herzustellen. Um nach dem Zusammenstecken der beiden Verbinderteile ein Herausrutschen derselben unter Zugbelastung zu vermeiden, ist der männliche Steckverbinderteil von einer ovalen Kupplungshülse umgeben, die auf dem langgestreckten Steckerkörper des männlichen Steckverbinderteils drehbar befestigt ist Die ovale, aus elastischem Material bestehende Kupplungshülse ist im Bereich des freien Endes des lang gestrecken Steckerkörpers mit einem Paar verklinkter Ausparungen versehen, die nach dem Zusammenschieben der beiden Verbinderteile in entsprechende Rastnasen einrasten, wenn die beiden Verbinderteile vollständig zusammen geschoben sind. Durch einen Druck in Richtung senkrecht auf die Verbindungslinie der beiden Rastnasen lässt sich die elastische Hülse aufgrund ihrer ovalen Ausgestaltung im Bereich der Rastnasen aufweiten, wodurch die Rastnasen nicht mehr in die Aussparungen eingreifen und der männliche Steckverbinderteil in axialer Richtung aus dem weiblichen Aufnahmeverbinderteil herausgezogen werden kann. Die Schritt gibt keinen Hinweis, die elastische ovale Hülse mit einem ersten und einem zweiten halbschalenartigen Abschnitt zu versehen und das zugrundeliegende Kernprinzip auf einen Adapter zum Anschluss einer respiratorischen Hilfseinrichtung für den menschlichen Körper zu übertragen.

Weiterhin zeigt die WO 2007/029199 A2 einen Anschlussadapter zum Anschluss einer respiratorischen Hilfseinrichtung an eignen Tubus, der zwei flexible Klemmabschnite aufweist, die an ihren Enden Rastnasen oder Vorsprünge aufweisen, welche in einander gegenüberliegende und parallel zu einander verlaufende Nuten am konischen Anschlusskonnektor einrasten, wenn der Anschlussadapter auf den Anschlusskonnektor aufgeschoben wird. Um den Adapter nach dem Aufschieben auf den Anschlusskonnektor wieder lösen zu können, wird der gesamte Anschlussadapter im Urzeigersinn verdreht, wodurch die Vorsprünge über die seitlichen Ränder der Nuten hinaus auf einem radial vergrößerten Abschnitt des Anschlusskonnektors verdreht werden und auf der kantenlosen Außenfläche des Anschlusskonnektors in axialer Richtung entlang gleiten können. Die Steckverbindung lässt sich somit nicht durch Anwenden einer Druckkraft sondern durch Verdrehen des Adapters lösen.

Demgemäß ist es eine Aufgabe der Erfindung, einen Adapter zum Anschluss von insbesondere respiratorischen Hilfseinrichtungen an den Anschlusskonnektor eines in den menschlichen Körper einsetzbaren Tubus zu schaffen, bei welchem sich die dichtende Klemmverbindung zwischen Adapter und Anschlusskonnektor unter Ausschluss von Drehmomenten oder Zugkräften auf den Tubus lösen lässt.

Diese Aufgabe wird erfingdungsgemäß durch die Merkmale von Anspruch 1 gelöst.

Unter dem nachfolgend aus Gründen der sprachlichen Vereinfachung verwendeten Begriff Tubus sind dabei allgemein medizinische Schläuche oder schlauchartige Objekt - wie z.B. Trachealtuben, Trachealkanülen oder auch Infusionskanülen etc. - zu verstehen, die zumindest zeitweilig in den menschlichen Körper eingesetzt werden, um einem Patienten. Atemluft, Flüssigkeiten, Medikamente oder Geräte wie Endoskope und dergleichen zuzuführen, bzw. um Körperflüssigkeiten vom Patienten abzuführen.

Durch die Erfindung ergibt sich der Vorteil, dass sich insbesondere die zuvor erwähnten respiratorischen Hilfseinrichtungen in sehr kurzer Zeit auch von ungeschultem Personal ohne eine nennenswerte Belastung des Patienten an einen in den menschlichen Körper eingesetzten Tubus, z.B. eine Trachealkanüle oder einen Trachealtubus, anschließen und von diesem wieder entfernen lassen.

Nach einer bevorzugten Ausgestaltung der Erfindung umfasst der Anschlussadapter einen äußeren hülsenartigen Abschnitt, der beispielsweise als Hülse aus Kunststoffmaterial, z.B. Polymethylen, ausgestaltet ist und eine Wandstärke von z.B. 1 mm bis 2 mm besitzen kann. Die Flexibilität des Materials ist hierbei in erfindungsgemäßer Weise derart gewählt, dass dieser im Querschnitt betrachtet ringartige Abschnitt durch Zusammendrücken im Bereich zweier diametral auf der Außenseite des Abschnitts vorgesehener Druckflächen mit dem Daumen und dem Zeigefinger zu einem Oval verformt werden kann.

Bevorzugt senkrecht, d. h. um 90° verdreht zu den Angriffspunkten der Druckkraft auf die Außenumfangsfläche des hülsenartigen Abschnitts, ist an dessen Innenfläche bevorzugt jeweils ein Verbindungselement angeordnet, das beispielsweise die Form eines sich radial nach innen erstreckenden Steges besitzen kann. Die Verbindungselemente sind an ihrem radial innen liegenden Ende jeweils mit einem halbschalenartigen Abschnitt versehen, der eine im Querschnitt halbringartige Form besitzt.

Die beiden halbschalenartigen Abschnitte weisen dabei zusammen betrachtet die Form einer in Längsrichtung zentral geteilten Hülse, bzw. eines Kegelstumpfes auf, dessen Innenflächen der Form der Außenfläche des Anschlusskonnektors in der Weise angepasst ist, dass der durch die halbschalenartigen Abschnitte definierte, im Querschnitt ringartige Klemmabschnitt einen geringfügig kleineren Innendurchmesser besitzt, als der Außendurchmesser des Anschlusskonnektors, wenn keine Druckkraft auf den äußeren hülsenartigen Abschnitt ausgeübt wird. Hierbei wird die Anlagekraft, mit der die beiden halbschalenartigen Abschnitte gegen die Außenfläche des Anschlusskonnektor gedrängt werden, bevorzugt allein durch die Eigensteifigkeit des flexiblen Materials des äußeren hülsenartigen Abschnitts erzeugt.

Wenn der äußere halbschalenartige Abschnitt, der bevorzugt die Querschnittsform eines Rings besitzt, durch eine äußere, diametral wirkende Druckkraft in ein Oval verformt wird, bewegen sich die beiden halbschalenartigen Abschnitte über die Stege betätigt z.B. um 1 bis 2 mm radial nach außen und erweitern hierdurch den Abstand zwischen den Innenflächen der beiden halbschalenartigen Abschnitte, wodurch die dichtende Klemmverbindung zwischen den halbschaligen Abschnitten und der Außenfläche des Anschlusskonnektors gelöst wird und der Adapter mit der zugehörigen respiratorischen Hilfseinrichtung vom Anschlusskonnektor abgezogen werden kann.

Um den Adapter mit der z.B. daran angeordneten respiratorischen Hilfseinrichtung auf den Anschlusskonnektor aufzustecken, wird der äußere hülsenartige Abschnitt zuerst mit dem Daumen und dem Zeigefinger diametral zusammen gedrückt, wodurch sich der äußere hülsenartigen Abschnitt im Querschnitt zu einem Oval verformt und die Innenflächen der beiden halbschalenartigen Abschnitte voneinander weg bewegt werden. Im Anschluss daran wird der Adapter in axialer Richtung auf den Anschlusskonnektor aufgeschoben und die Druckkraft gelöst, wodurch sich der äußere hülsenartige Abschnitt aufgrund der Eigenelastizität des Materials in seine im Querschnitt ringförmige Ausgangsposition zurück bewegt und die Innenflächen der beiden halbschalenartigen Abschnitte über die Verbindungselemente gegen die Außenfläche des Anschlusskonnektors bewegt werden und diese dichtend klemmen.

Nach einer weiteren Ausführungsform der Erfindung umfasst der Klemmabschnitt des Anschlussadapters zwei Halbschalen oder halbschalenartige Abschnitte mit einer Länge von z. B. 3 - 4 cm, die in etwa in ihrer Mitte über beiderseits angeordnete elastische Stege verbunden sind. Die beiden Stege definieren hierbei eine Schwenkachse, die bewirkt, dass sich der dem Anschlusskonnektor zugewandte Teil des aufweitbaren Abschnitts nach Art eines Entenschnabels erweitert, wenn jenseits der Stege auf die dem Abschlusskonnektor abgewandte Seite der beiden Halbschalen eine die Halbschalen gegeneinander drückende Druckkraft ausgeübt wird.

Nach einer Weiterbildung dieser Ausführungsform der Erfindung kann zur Erhöhung der Klemmkraft im Bereich des dem Anschlusskonnektor abgewandten Endes ein weiteres federelastisches Element vorgesehen sein, welches zwischen den beiden halbschalenartigen Abschnitten wirkt und diese auseinander drängt. Das federelastische Element ist hierbei bevorzugt als zusätzlicher Steg aus elastischem Material ausgestaltet, der in vorteilhafter Weise dafür sorgt, dass beim Einsatz des Adapters in Verbindung mit einer künstlichen Nase der Schaumstofffilter für die Rückbefeuchtung der eingeatmeten Atemluft in seiner Position fixiert wird.

Um bei der zuletzt beschriebenen Ausführungsform der Erfindung die beiden halbschalenartigen Abschnitte in der Klemmenposition gegen einen Austritt von Atemluft abzudichten, kann es weiterhin vorgesehen sein, die beiden halbschalenartigen Abschnitte im Bereich der seitlich verbleibenden Schlitze mit Dichtungsmitteln, z.B. innenliegenden Dichtlippen oder mit einer Umhüllung aus flexiblem Material versehen sind, welch letztere beispielsweise aus einem dünnenwandigen elastischen Kunststoffmaterial oder einer elastischen Folie bestehen kann.

Die Erfindung wird nachfolgend mit Bezug auf die Zeichnungen beispielhaft in Verbindung mit einem Tubus in Form einer Trachealkanüle anhand von zwei bevorzugten Ausführungsformen beschrieben.

In den Zeichnungen zeigen:
- Fig. 1a: eine schematische seitliche Querschnittsansicht einer Trachealkanüle mit einem aufgesetzten Adapter nach einer ersten Ausführungsform der Erfindung in der Klemmposition,
- Fig. 1b: eine teilweise transparente Frontansicht des Adapters von Fig. 1b,
- Fig. 2a: eine schematische seitliche Querschnittsansicht der Trachealkanüle mit dem aufgesetzten Adapter nach der ersten Ausführungsform in der Freigabeposition,
- Fig. 2b: eine teilweise transparente Frontansicht des Adapters von Fig. 2a,
- Fig. 3a: eine schematische seitliche Querschnittsansicht einer Trachealkanüle mit einem aufgesetzten Adapter nach einer weiteren Ausführungsform der Erfindung in der Klemmposition,
- Fig. 3b: eine teilweise transparente Frontansicht des Adapters von Fig. 3a,
- Fig. 4a: eine schematische seitliche Querschnittsansicht der Trachealkanüle mit dem aufgesetzten Adapter nach der zweiten Ausführungsform in der Freigabeposition, und
- Fig. 4b: eine teilweise transparente Frontansicht des Adapters von Fig. 4a.

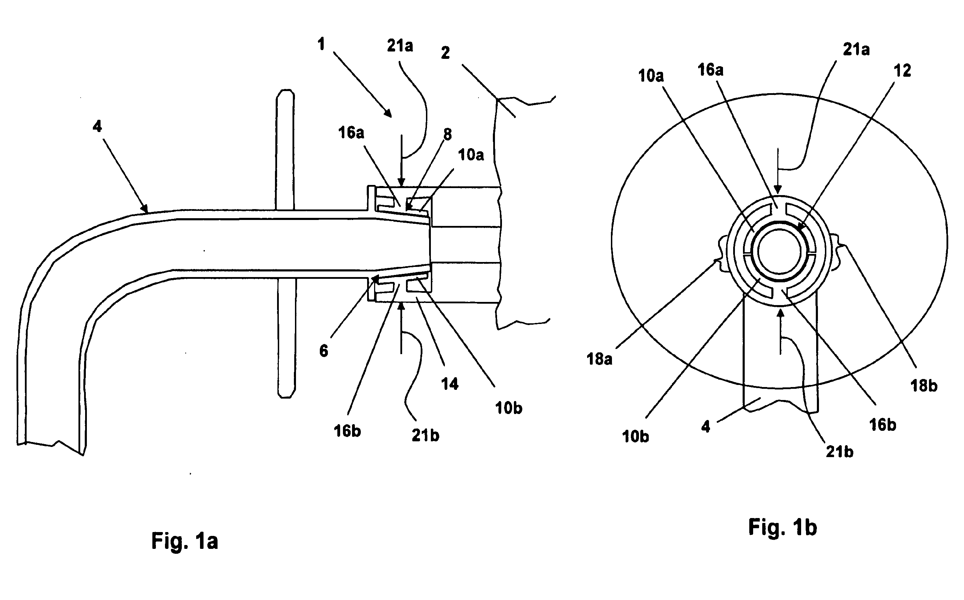

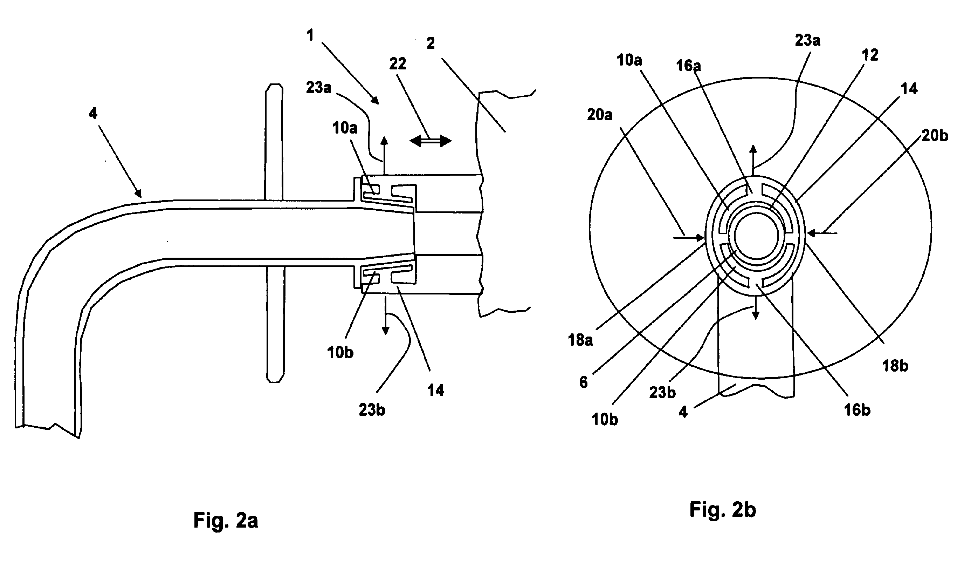

Wie in Fig. 1a und 1b gezeigte ist, umfasst ein erfindungsgemäßer Anschlussadapter 1 zum Anschluss von in den Zeichnungen lediglich schematisch angedeuteten respiratorischen Hilfseinrichtungen 2 wie Schläuchen, Tubus-Verlängerungen, Filtern, Beatmungsbeuteln oder künstlichen Nasen etc. an einen Tubus in Form einer beispielhaft gezeigten Trachealkanüle 4, die einen sich konisch verjüngenden Anschlusskonnektor 6 besitzt, einen Klemmabschnitt 8, der durch Anwendung einer auf den Klemmabschnitt 8 wirkenden Druckkraft aus der in Fig. 1a, 1b gezeigten ersten Klemmposition - in der der Innendurchmesser des Klemmabschnitts 8 kleiner ist als der Außendurchmesser des Anschlusskonnektors 6 - radial in eine zweite, in Fig. 2a, 2b gezeigte Freigabeposition aufgeweitet werden kann.

Wie in Fig. 1a und 1b im Detail gezeigt ist, umfasst der Klemmabschnitt 8 einen ersten und zweiten halbschalenartigen Abschnitt 10a, 10b, der in der ersten Klemmposition unter Bildung eines im Wesentlichen ringförmigen Querschnitts an der Außenfläche 12 des Anschlusskonnektors 6 anliegt. Die halbschalenartigen Abschnitte 10a, 10b befinden sich dabei innerhalb eines äußeren hülsenartigen Abschnitts 14 aus flexiblem Material, mit dem sie über ein ersteres und zweites Verbindungselement in Form eines Verbindungssteges 16a, 16b verbunden sind. Die durch die beiden Pfeile 21a und 21b angedeutete Klemmkraft, mit der die halbschalenartigen Abschnitte 10a, 10b unter Vorspannung am Anschlusskonnektor 6 anliegen, wird dabei durch die Eingenelastizität des Materials erzeugt.

Jeder der beiden bevorzugt integral mit dem äußeren hülsenartigen Abschnitt 14 gebildeten Verbindungsstege 16a, 16b entspringt dabei in erfindungsgemäßer Weise im Bereich der inneren Fläche des äußeren hülsenartigen Abschnitts 14 an einer Position, die gegenüber einer auf der Außenseite des äußeren hülsenartigen Abschnitts 14 vorgesehenen Druckfläche 18a, 18b um 90° versetzt angeordnet ist und erstreckt sich von dort aus radial nach innen.

Wie anhand der Querschnittsdarstellung von Fig. 2b zu erkennen ist, wird durch Anwenden einer durch die Pfeile 20a, 20b angedeuteten Druckkraft, die z.B. durch den Daumen und den Zeigefinger einer menschlichen Hand aufgebracht werden kann, der äußere hülsenartige Abschnitt 14 diametral zu einem Oval verformt, was dazu führt, dass die halbschalenartigen Abschnitte 10a und 10b über die Verbindungsstege 16a, 16b in Richtung der Pfeile 23a und 23b voneinander weg bewegt werden, wodurch sich die Klemmverbindung löst. In dieser Position der halbschalenartigen Abschnitte 10a, 10b kann der Adapter 1 im Anschluss daran in Richtung des Pfeils 22 vom Anschlusskonnektor 6 abgezogen, bzw. auf diesen aufgesteckt werden, wie dies in Fig. 2a angedeutet ist.

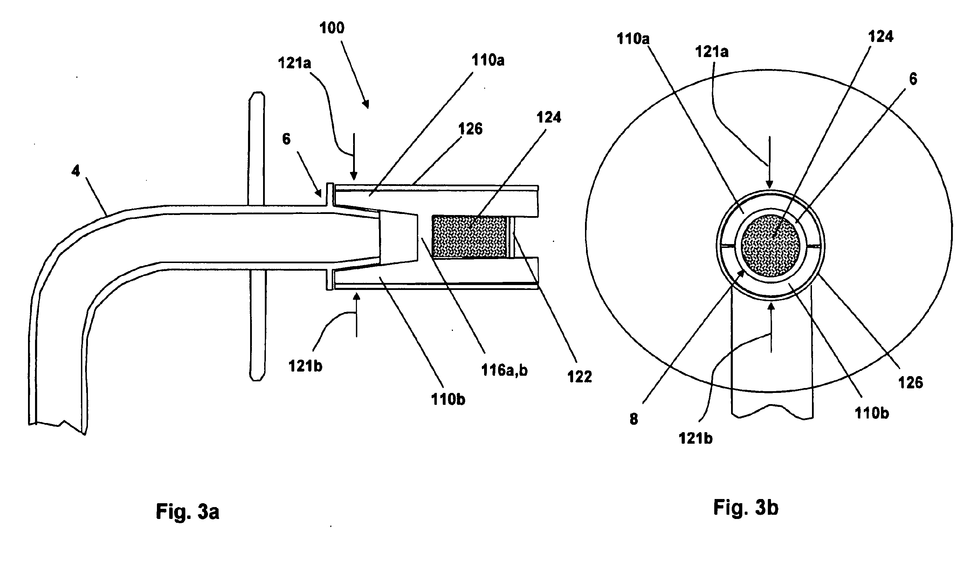

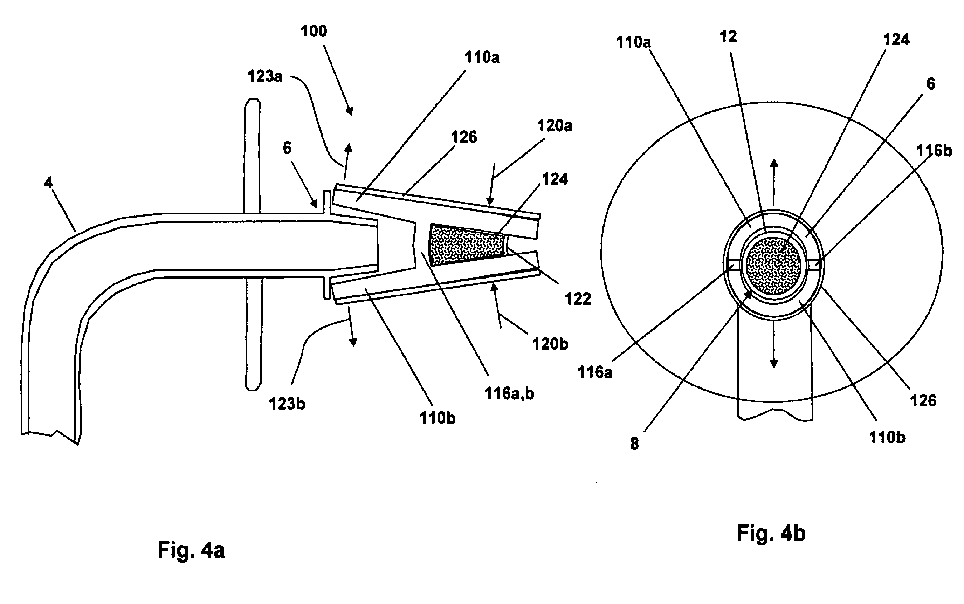

Wie in Fig. 3a, 3b und 4a, 4b dargestellt ist, kann es gemäß einer weiteren Ausführungsform der Erfindung vorgesehen sein, dass der in diesem Falle mit der Bezugsziffer 100 bezeichnete Adapter einen ersten und zweiten über elastische Stege 116a, 116b miteinander verbundenen halbschalenartigen Abschnitt 110b und 110b aufweist.

Wie sich der Darstellung von Fig. 4a und 4b weiterhin entnehmen lässt, definieren Sie beiden Stege 116a, 116b aufgrund ihrer Eigenelastizität eine Art Schwenkachse, die bewirkt, dass sich der dem Anschlusskonnektor 6 zugewandte Teil des aufweitbaren Abschnitts oder Klemmabschnitts 8 nach Art eines Entenschnabels erweitert, wenn jenseits der beiden Stege 116a, 116b auf die dem Abschlusskonnektor 6 abgewandte Seite der beiden halbschalenartigen Abschnitte 110a und 110b eine durch die Pfeile 120a, 120b angedeutete Druckkraft ausgeübt wird. Durch diese Druckkraft werden die beiden halbschalenartigen Abschnitte 110a und 110b, die gemäß der Darstellung von Fig. 3a und 3b in der Klemmposition mit einer durch die Pfeile 121a und 121b angedeuteten Klemmkraft unter Vorspannung am Anschlusskonnektor 6 anliegen, in Richtung der Pfeile 123a und 123b vom Anschlusskonnektor 6 wegbewegt.

Nach einer Weiterbildung dieser Ausführungsform der Erfindung kann zur Erhöhung der Klemmkraft im Bereich des dem Anschlusskonnektor 6 abgewandten Endes ein zusätzliches federelastisches Element 122 vorgesehen sein, welches zwischen den beiden halbschalenartigen Abschnitten 110a und 110b eine Druckkraft erzeugt, welche die Abschnitte 110a und 110b auseinander drängt. Das federelastisches Element 122 ist hierbei bevorzugt als zusätzlicher Steg aus elastischem Material ausgestaltet, der in vorteilhafter Weise dafür sorgt, dass beim Einsatz des Adapters 100 in Verbindung mit einer in Fig. 3 und 4 ebenfalls angedeuteten künstlichen Nase der Schaumstofffilter 124 für die Rückbefeuchtung der eingeatmeten Luft in seiner Position verbleibt.

Der erfindungsgemäße Adapter 1, 100 mit seinen zugehörigen Komponenten wird bevorzugt in einem Stück, insbesondere als Spritzgussteil gefertigt, obgleich es ebenfalls vorgesehen sein kann, dass der Adapter 1, 100 aus mehreren Einzelkomponenten zusammengesetzt wird. So ist es beispielsweise denkbar, dass der in den Figuren 3 und 4 gezeigte Adapter 100 durch nachträgliches Schlitzen einer auf die gewünschte Länge zugeschnittenen Kunststoffhülse erhalten wird, welche zur Abdichtung der Schlitze im Bereich des Anschlusskonnektors 6 mit entsprechenden Dichtelementen wie Dichtlippen oder einer in den Figuren 3 und 4 angedeuteten Umhüllung 126 versehen sein kann, welch letztere bevorzugt aus einem flexiblen, z.B. gummiartigen Werkstoff besteht.

Obgleich die Erfindung zuvor anhand eines männlichen Anschlusskonnektors 6 in Verbindung mit einem weiblichen Adapter 1, 100 beschrieben wurde, welcher bevorzugt unmittelbar an einer respiratorischen Hilfseinrichtung 2 angeordnet ist, versteht es sich, dass der der Erfindung zu Grunde liegende Gedanke auch auf die umgekehrte Konfiguration bestehend aus einem männlichen Adapter und einem weiblichen Anschlusskonnektor anwendbar ist.

### Liste der Bezugszeichen

- 1: erfindungsgemäßer Anschlussadapter
- 2: respiratorische Hilfseinrichtung
- 4: Trachealkanüle
- 6: Anschlusskonnektor
- 8: Klemmabschnitt
- 10a: erster halbschalenartiger Abschnitt
- 10b: zweiter halbschalenartiger Abschnitt
- 12: Außenfläche des Anschlusskonnektors
- 14: äußerer hülsenartiger Abschnitt
- 16a: erster Verbindungssteg
- 16b: zweiter Verbindungssteg
- 18a: erste Druckfläche
- 18b: zweiter Druckfläche
- 20a: Pfeil
- 20b: Pfeil
- 21a: Pfeil, der Klemmkraft andeutet
- 21b: Pfeil, der Klemmkraft andeutet
- 22: Pfeil
- 23a: Pfeil, der die Bewegung des ersten halbschalenartigen Abschnitts beim Lösen der Klemmkraft andeutet
- 23b: Pfeil, der die Bewegung des zweiten halbschalenartigen Abschnitts beim Lösen der Klemmkraft andeutet
- 100: Adapter
- 110a: erster halbschalenartiger Abschnitt
- 110b: zweiter halbschalenartiger Abschnitt
- 116a: erster elastischer Steg
- 116b: zweiter elastischer Steg
- 120a: Pfeil
- 120b: Pfeil
- 121a: Pfeil, der Klemmkraft andeutet
- 121b: Pfeil, der Klemmkraft andeutet
- 122: federelastisches Element
- 123a: Pfeil, der die Bewegung des ersten halbschalenartigen Abschnitts beim Lösen der Klemmkraft andeutet
- 123b: Pfeil, der die Bewegung des zweiten halbschalenartigen Abschnitts beim Lösen der Klemmkraft andeutet
- 124: Schaumstofffilter
- 126: Umhüllung

## Patentansprüche

1. Adapter (1, 100) zum Anschluss einer insbesondere respiratorischen Hilfseinrichtung (2) an einen Tubus (4) mit einem sich bevorzugt konisch verjüngenden Anschlusskonncktor (6), wobei der Adapter einen Klemmabschnitt (8) umfasst, der reibschlüssig am Anschlusskonnektor (6) fixierbar ist, und der Klemmabschnitt (8) durch Anwendung einer auf den Klemmabschnitt wirkenden Druckkraft aus einer ersten Klemmposition, in der der Innendurchmesser des Klemmabschnitt (8) kleiner ist als der Außendurchmesser des Anschlusskonnektors (6) radial in eine zweite Freigabeposition aufweitbar ist, in der der Innendurchmesser des Klemmabschnitt (8) größer ist als der Außendurchmesser des Anschlusskonnektors (6),
**dadurch gekennzeichnet, dass**
der Klemmabschnitts (8) einen ersten und zweiten halbschalenartigen Abschnitt (10a, 10b; 110a, 110b) aufweist, der in der ersten Klemmposition an der Außenfläche des Anschlusskonnektors (6) anliegt und der in der zweiten Freigabeposition zum Lösen der Klemmverbindung im Abstand von der Außenfläche des Anschlusskonnektors (6) angeordnet ist.

2. Adapter nach Anspruch 1,
**dadurch gekennzeichnet, dass**
die halbschalenartigen Abschnitte (10a, 10b) innerhalb eines äußeren hülsenartigen Abschnitts (14) aus flexiblem Material angeordnet sind, und dass wenigstens einer der halbschalenartigen Abschnitte (10a, 10b) mit dem äußern hülsenartigen Abschnitt (14) über ein Verbindungselement (16a, 16b) verbunden ist, derart, dass die halbschalenartigen Abschnitte (10a, 10b) bei der Einwirkung einer Druckkraft auf den äußeren hülsenartigen Abschnitt (14) voneinander wegbewegt werden.

3. Adapter nach Anspruch 2,
**dadurch gekennzeichnet, dass**
zwei halbschalenartigen Abschnitte (10a, 10b) vorgesehen sind, die jeweils über einen sich innerhalb des äußern hülsenartigen Abschnitts (14) radial nach innen erstreckenden Steg (16a, 16b) mit dem äußeren hülsenartigen Abschnitt (14) gekoppelt sind, wobei jeder der beiden Stege (16a, 16b) um 90° versetzt zu einer ersten und zweiten diametral auf der Außenseite des äußeren hülsenartigen Abschnitts vorgesehenen Druckfläche (18a, 18b) angeordnet ist, über welche sich der äußere hülsenartige Abschnitt (14) deformieren lässt.

4. Adapter nach Anspruch 1,
**dadurch gekennzeichnet, dass**
der erste und zweite halbschalenartige Abschnitt (110a, 110b) über beiderseits angeordnete, eine Schwenkachse definierende elastische Stege (116a, 116b) verbunden sind, derart, dass der dem Anschlusskonnektor (6) zugewandte Teil des ersten und zweiten halbschalenartigen Abschnitts (110a, 110b) voneinander weg aus der ersten Klemmposition in die zweite Freigabeposition verschwenkt werden, wenn die halbschalenartigen Abschnitte (110a, 110b) im Bereich ihrer dem Anschlusskonnektor (6) gegenüberliegenden Enden mit einer Druckkraft beaufschlagt werden.

5. Adapter nach Anspruch 4,
**dadurch gekennzeichnet, dass**
im Bereich des dem Anschlusskonnektor (6) abgewandten Endes ein zwischen dem ersten und zweiten halbschalenartigen Abschnitt (110a, 110b) wirkendes foderelastisches Element (122) angeordnet ist.

6. Adapter nach Anspruch 4 oder 5,
**dadurch gekennzeichnet, dass**
die halbschalenartigen Abschnitte (110a, 110b) durch Dichtmittel, insbesondere eine flexible Umhüllung (126), gegen einen Austritt von Atemluft abgedichtet werden.

7. Hifseinrichtung, insbesondere respiratorische Hilfseinrichtung (2) wie Schlauch, Tubus-Verlängerung, Atemluftfilter, Beatmungsbeutel oder künstlichen Nase,
**gekennzeichnet durch**
einen Adapters (1, 100) nach einem der vorhergehenden Ansprüche.

## Claims

1. Adapter (1, 100) for joining an in particular respiratory accessory (2) to an airway (4) having a preferably conically tapered joint connector (6), wherein the adaptor comprises a clamping section (8) which can be frictionally engaged with the joint connector (6) and can be widened from a first clamping position, in which the internal diameter of the clamping section (8) is smaller than the external diameter of the joint connector (6), radially into a second release position, in which the internal diameter of the clamping section (8) is greater than the external diameter of the joint connector (6) by applying a pressure force acting on the clamping section,
**characterised in that**
the clamping section (8) comprises a first and second half-shell like section (10a, 10b; 110a, 110b), abutting against the external surface of the joint connector (6) in the first clamping position, and positioned at a distance from the external surface of the joint connector (6) in the second release position for opening the clamping connection.

2. Adapter according to claim 1,
**characterised in that**
the half-shell like sections (10a, 10b) are arranged within an outer sleeve-like section (14) consisting of a flexible material, and **in that** at least one of the half-shell like sections (10a, 10b) are connected with the outer sleeve-like section (14) via a connection element (16a, 16b) in such a way that the half-shell like sections (10a, 10b) are moved away from each other towards the outer sleeve-like section (14) under action of a pressure force.

3. Adapter according to claim 2,
**characterised in that**
two half-shell like sections (10a, 10b) are envisaged, each coupled with an outer sleeve-like section (14) radially extending inwards across a bridge (16a, 16b) within said outer sleeve-like section (14), whereby each of the two bridges (16a, 16b) is arranged offset by 90° from a first and second pressure surface (18a, 18b) envisaged diametrically on the outside of the outer sleeve-like section, across which the outer sleeve-like section (14) can be deformed.

4. Adaptor according to claim 1,
**characterised in that**
the first and second half-shell sections (110a, 110b) are connected via elastic bridges (116a, 116b) located on both sides and defining a pivot axis in such a way that the parts of the first and second half-shell like sections (110a, 110b) facing the joint connector (6) are tilted away from each other from the first clamping position into the second release position when the half-shell like sections (110a, 110b) are subjected to a pressure force in the area of their ends opposite the joint connector (6).

5. Adapter according to claim 4,
**characterised in that**
a spring elastically acting element (122) is located between the first and second half-shell like section (110a, 110b) in the area of the end facing away from the joint connector (6).

6. Adapter according to claim 4 or 5,
**characterised in that**
the half-shell like sections (110a, 110b) are sealed by means of a sealant, in particular a flexible encasing (126), against an escape of breathing air.

7. Accessory, in particular a respiratory accessory (2) such as a hose, airway extension, breathing air filter, bag valve mask or artificial nose,
**characterised by**
an adaptor (1, 100) according to one of the preceding claims.

## Revendications

1. Adaptateur (1, 100) destiné à un raccordement d'un appareil auxiliaire (2), en particulier respiratoire, à un tube (4) avec un connecteur de raccordement (6) se rétrécissant de préférence de manière conique, l'adaptateur comprenant une section de serrage (8) qui peut être fixée par frottement au connecteur de raccordement (6), et la section de serrage (8) pouvant être élargie radialement à partir d'une première position de serrage dans laquelle le diamètre intérieur de la section de serrage (8) est inférieur au diamètre extérieur du connecteur de raccordement (6) jusqu'à une seconde position de libération dans laquelle le diamètre intérieur de la section de serrage (8) est supérieur au diamètre extérieur du connecteur de raccordement (6) grâce à l'application d'une force de compression agissant sur la section de serrage,
**caractérisé en ce que**
la section de serrage (8) présente des première et seconde sections (10a, 10b; 110a, 110b) de type demi-coque, qui, dans la première position de serrage, reposent sur la surface extérieure du connecteur de raccordement (6) et qui, dans la seconde position de libération, sont agencées à une distance de la surface extérieure du connecteur de raccordement (6) en vue du desserrage de la liaison par serrage.

2. Adaptateur selon la revendication 1,
**caractérisé en ce que**
les sections (10a, 10b) de type demi-coque sont agencées au sein d'une section (14) extérieure de type manchon en matériau flexible, et au moins une des sections (10a, 10b) de type demi-coque est reliée par l'intermédiaire d'un élément de liaison (16a, 16b) à la section (14) extérieure de type manchon, de sorte que les sections (10a, 10b) de type demi-coque sont éloignées l'une de l'autre lors de l'action d'une force de compression sur la section (14) extérieure de type manchon.

3. Adaptateur selon la revendication 2,
**caractérisé en ce que**
deux sections (10a, 10b) de type demi-coque sont prévues, qui sont couplées à la section (14) extérieure de type manchon respectivement par l'intermédiaire d'une traverse (16a, 16b) s'étendant radialement vers l'intérieur au sein de la section (14) extérieure de type manchon, chacune des deux traverses (16a, 16b) étant agencée décalée de 90° par rapport à des première et seconde surfaces de pression (18a, 18b) prévues diamétralement sur le côté extérieur de la section extérieure de type manchon, par l'intermédiaire desquelles la section (14) extérieure de type manchon peut être déformée.

4. Adaptateur selon la revendication 1,
**caractérisé en ce que**
les première et seconde sections (110a, 110b) de type demi-coque sont reliées par l'intermédiaire de traverses (116a, 116b) élastiques définissant un axe de pivotement, agencées bilatéralement, de sorte que les parties, tournées vers le connecteur de raccordement (6), des première et seconde sections (110a, 110b) de type demi-coque s'écartent l'une de l'autre en pivotant à partir de la première position de serrage jusque dans la seconde position de libération lorsque les sections (110a, 110b) de type demi-coque sont soumises à une force de compression dans le secteur de leurs extrémités situées vis-à-vis du connecteur de raccordement (6).

5. Adaptateur selon la revendication 4,
**caractérisé en ce que**
un élément (122) à élasticité de ressort agissant entre les première et seconde sections (110a, 110b) de type demi-coque est agencé dans le secteur de l'extrémité détournée du connecteur de raccordement (6).

6. Adaptateur selon la revendication 4 ou 5,
**caractérisé en ce que**
les sections (110a, 110b) de type demi-coque sont rendues étanches à une entrée d'air de respiration grâce à un moyen d'étanchéité, en particulier une enveloppe flexible (126).

7. Appareil auxiliaire, en particulier appareil auxiliaire respiratoire (2), comme un tube, un prolongateur de tube, un filtre à air de respiration, un insufflateur ou des nez artificiels,
**caractérisé par**
un adaptateur (1, 100) selon l'une quelconque des revendications précédentes.
